Europäisches Patentamt

European Patent Office

Office européen des brevets

(19)

(11) Veröffentlichungsnummer: **0 283 929 B1**

(12) **EUROPÄISCHE PATENTSCHRIFT**

(45) Veröffentlichungstag der Patentschrift: 04.09.91

(51) Int. Cl.⁵: **A61F 9/00**

(21) Anmeldenummer: 88104237.8

(22) Anmeldetag: 17.03.88

(54) **Handgriff mit einer Doppelkanüle.**

(30) Priorität: 25.03.87 DE 8704459 U

(43) Veröffentlichungstag der Anmeldung:
28.09.88 Patentblatt 88/39

(45) Bekanntmachung des Hinweises auf die
Patenterteilung:
04.09.91 Patentblatt 91/36

(84) Benannte Vertragsstaaten:
CH DE FR GB IT LI NL

(56) Entgegenhaltungen:
US-A- 3 809 093
US-A- 3 867 947
US-A- 3 937 222

(73) Patentinhaber: Erbe Elektromedizin GmbH.
Waldhörnlestrasse 17
W-7400 Tübingen(DE)

(72) Erfinder: van Gerven, Johannes Theodorus
Maria
Eugenstrasse 10
W-7403 Ammerbuch 1(DE)

(74) Vertreter: Endlich, Fritz, Dipl.-Phys.
Postfach 1326 Blumenstrasse 8
W-8034 Germering(DE)

**Beschreibung**

Die Neuerung betrifft einen Handgriff mit einer Doppelkanüle zum Absaugen von Linsentrümmern bei der Durchführung von Linsenstar-Operationen.

Es sind bereits Linsen-Absauggeräte für medizinische Zwecke bekannt, für die ein oder mehrere derartige Handgriffe mit einer Doppelkanüle zum Absaugen von Linsentrümmern vorgesehen sind (DE-GM 84 37 836), wobei die Doppelkanüle aus zwei koaxialen Röhrchen besteht, wovon das innere Röhrchen eine geschlossene Spitze und eine seitlich angeordnete Saugöffnung aufweist. Das vordere Ende des äußeren Röhrchens ist in einem Abstand von der Spitze mit dem inneren Röhrchen verbunden und weist zwei seitlich angeordnete Spülöffnungen auf, die vorzugsweise in Umfangsrichtung der Doppelkanüle relativ zu der Saugöffnung zumindest angenähert um 45° versetzt angeordnet sind.

Üblicherweise findet in Verbindung mit einem Absauggerät ein Satz von derartigen Handgriffen Verwendung, wobei die Saugöffnung am vorderen Ende der inneren Röhrchen der Handgriffe einen unterschiedlichen Durchmesser aufweist, so daß bei Durchführung einer Operation zunächst größere und dann kleinere Teilchen von Linsentrümmern abgesaugt werden können. Bei den meisten Operationen werden zwei oder drei Handgriffe dieser Art mit einer unterschiedlichen Saugöffnung benutzt. Ferner findet bei der Durchführung einer Operation mit einem derartigen Absauggerät noch eine Polierkanüle Verwendung, die mit Diamantpulver beschichtet ist, um durch Polieren der hinteren Kapsel zu verhindern, daß sekundäre Katarakte verursacht werden können.

Es ist Aufgabe der Neuerung, einen Handgriff dieser Art derart zu gestalten, daß einerseits bei der Durchführung einer Operation eine Zeitersparnis erzielbar ist, und daß andererseits die Operation weniger traumatisch durchgeführt werden kann.

Diese Aufgabe wird bei einem Handgriff der eingangs genannten Art erfindungsgemäß dadurch gelöst, daß auf der der Saugöffnung gegenüberliegenden Seite eine Polierfläche vorgesehen ist. Bei einer derartigen Ausgestaltung können beim Polieren abgeriebene Teilchen einerseits sofort abgesaugt werden. Eine derartige Polierfläche stört nicht beim normalen Arbeiten. Andererseits wird für den Chirurgen eine Zeitersparnis erzielt und vor allem für den Patienten die Verletzungsgefahr verringert, weil bei Benutzung eines Handgriffs gemäß der Erfindung zumindest einmal weniger eine Spitze eingeschoben und herausgezogen werden muß. Da eine zusätzliche Polierkanüle nicht mehr erforderlich ist, ergeben sich auch weitere Vorteile hinsichtlich Aufbewahrung und Herstellung eines Satzes von für eine Operation erforderlichen Instrumenten. Ferner ist die Ausbildung eines Irishäkchens an dem Handgriff mit der Polierfläche besonders zweckmäßig, weil die durch die Iris begrenzte Öffnung nur anfänglich mit Medikamenten vergrößert werden kann, so daß diese Öffnung nahe dem Ende der Operation wesentlich kleiner ist.

Anhand der Zeichnung soll die Neuerung beispielsweise näher erläutert werden. Es zeigen:

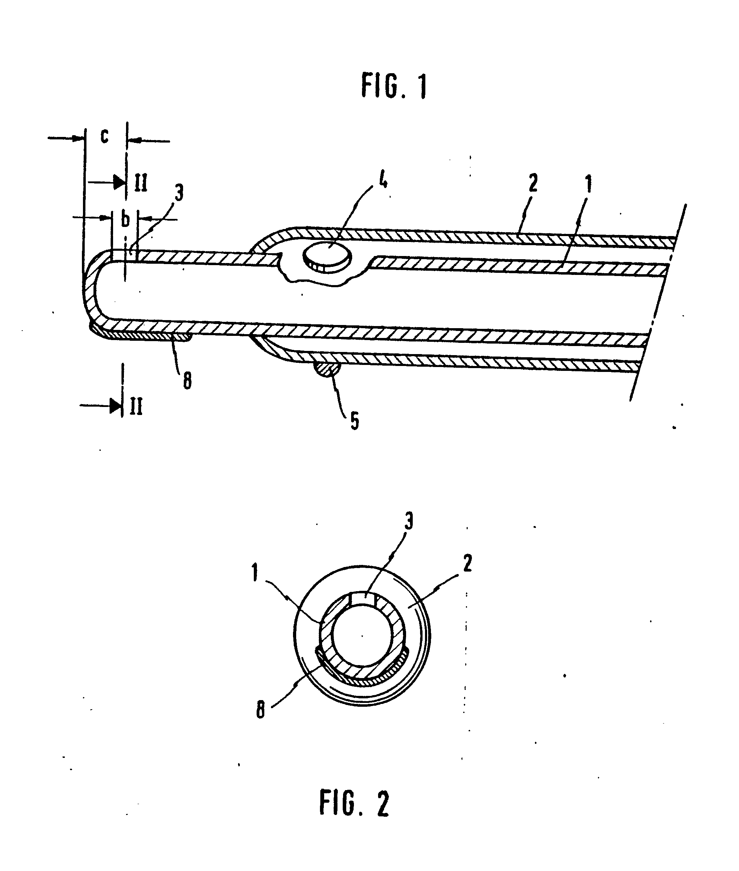

Fig. 1 und 2 Schnittansichten eines Ausführungsbeispiels gemäß der Neuerung.

Bei dem in Fig. 1 dargestellten Ausführungsbeispiel ist eine geradlinige Anordnung der beiden koaxial zueinander angeordneten Röhrchen 1 und 2 vorgesehen. Das innere Röhrchen 1 weist eine geschlossene Spitze auf, in der eine seitlich angeordnete Saugöffnung 3 ausgebildet ist. Der Abstand c in Fig. 1 kann beispielsweise 0,5 mm und der Durchmesser b 0,3 mm betragen. Im allgemeinen beträgt bei einem Satz von Handgriffen für die Durchführung einer Linsenstar-Operation der Abstand c zwischen 0,5 und 0,7 mm und der Durchmesser b der Saugöffnungen 3 mit unterschiedlicher Größe liegt zwischen 0,3 und 0,7 mm. Das äußere Röhrchen 2 ist angrenzend an sein vorderes Ende mit zwei Spülöffnungen 4 versehen, die vorzugsweise um 45° versetzt zu der Saugöffnung 3 angeordnet sind, und von denen nur eine in Fig. 1 sichtbar ist. Der Durchmesser der beiden Spülöffnungen beträgt bei dem dargestellten Ausführungsbeispiel 0,6 mm. Die Spitze des inneren Röhrchens 1 kann einen Abstand von dem vorderen Ende des äußeren Röhrchens zwischen 1,3 und 1,5 mm aufweisen.

Wenn die beiden Spülöffnungen 4 angenähert um 45° versetzt zu der Saugöffnung 3 sind, kann eine intensive Spülung in der Vorderkammer des Auges erzielt werden, weil beide Spülströme auf die Innenwand der hinteren Linsenkapsel gerichtet werden können, so daß entlang des verhältnismäßig kurzen Wegs zwischen den beiden Spülöffnungen 4 und der dazwischenliegenden Saugöffnung 3 beidseitig eine zu der Saugöffnung gerichtete Wirbelströmung erzielt werden kann, die eine intensive Spülung und damit eine bessere Entfernung der Linsentrümmer ermöglicht.

Gemäß der Neuerung ist auf der der Saugöffnung 3 gegenüberliegenden Seite eine Polierfläche 8 vorgesehen, die vorzugsweise durch eine Schicht aus aufgesintertem Diamantpulver gebildet ist. Zweckmäßigerweise ist auf der den beiden Spülöffnungen gegenüberliegenden Seite des äußeren Röhrchens 2 ein als Irishäkchen dienender Anschlag 5 ausgebildet, mit dem die Iris auseinandergezogen werden kann, wenn sie bei einer Operation nach einer gewissen Zeit wieder kleiner wird. Der in Fig. 1 dargestellte Handgriff ist derjenige Handgriff in einem Satz von normalerweise für die durchführung einer Operation benutzten Handgriffen, der die Saugöffnung 3 mit dem kleinsten Durchmesser oder der kleinsten Querschnittsfläche hat. Deshalb muß keine zusätzliche Polierkanüle in das Auge eingeführt werden, wodurch das Risiko der durch das Einschieben und Herausziehen gegebenen Verletzungsgefahr verringert wird.

Das vorragende Ende des inneren Röhrchens kann geradlinig oder abgebogen ausgebildet sein. Es ist ferner möglich, die Achse der beiden koaxialen Röhrchen 1,2 bogenförmig auszubilden, so daß ein besseres Eindringen in die Augenkammerwinkel erzielbar ist, da dann die Nase des Patienten dabei nicht stört.

Die Polierfläche 8 wirkt sich bei der normalen Arbeitsweise zum Absaugen von Linsentrümmern nicht störend aus, ermöglicht aber ohne weiteres ein Polieren und damit auch das Absaugen der dabei abgeriebenen Teilchen.

## Patentansprüche

1. Handgriff mit einer Doppelkanüle zum Absaugen von Linsentrümmern bei der Durchführung von Linsenstar-Operationen, wobei die Doppelkanüle aus zwei koaxialen Röhrchen besteht, wovon das innere Röhrchen eine geschlossene Spitze und eine seitlich angeordnete Saugöffnung aufweist, und das vordere Ende des äußeren Röhrchens in einem Abstand von der Spitze mit dem inneren Röhrchen verbunden ist und zwei seitlich angeordnete Spülöffnungen aufweist, **dadurch gekennzeichnet,** daß auf der der Saugöffnung (3) gegenüberliegenden Seite eine Polierfläche (8) vorgesehen ist.

2. Handgriff nach Anspruch 1, **dadurch gekennzeichnet, daß** die Polierfläche (8) durch aufgesintertes Diamantpulver gebildet ist.

3. Handgriff nach Anspruch 1 oder 2, **dadurch gekennzeichnet, daß** auf der den beiden Spülöffnungen (4) gegenüberliegenden Seite des äußeren Röhrchens (2) ein als Irishäkchen dienender Anschlag (5) ausgebildet ist.

4. Handgriff nach einem der vorhergehenden Ansprüche, **dadurch gekennzeichnet, daß** das vorragende Ende des inneren Röhrchens (1) geradlinig ausgebildet ist.

5. Handgriff nach einem der Ansprüche 1 bis 3, **dadurch gekennzeichnet, daß** das vorragende Ende des inneren Röhrchens (1) abgebogen ausgebildet ist.

6. Aus zwei oder mehr Handgriffen nach dem Oberbegriff des Anspruchs 1 bestehender Satz für die Durchführung einer Linsenstar-Operation, wobei die Saugöffnung am vorderen Ende der inneren Röhrchen eine unterschiedliche Größe aufweist, **dadurch gekennzeichnet, daß** der Handgriff mit der kleinsten Saugöffnung (3) auf der der Saugöffnung gegenüberliegenden Seite eine Polierfläche (8) aufweist.

## Claims

1. A hand tip with a double lumen for aspirating lenticular debris when performing lenticular cataract operations, with said double lumen consisting of two coaxial tubes, of which the inner tube comprises a closed point and a laterally disposed aspirating aperture, and the front end of the outer tube is connected to the inner tube at a distance from the tip and comprises two laterally disposed irrigating apertures, characterised in that a polished surface (8) is provided on the side opposite the aspirating aperture (3).

2. A hand tip according to Claim 1, characterised in that the polished surface (8) is formed by sintered-on diamond powder.

3. A hand tip according to Claim 1 or 2, characterised in that on the side of the outer tube (2) opposite the two irrigating apertures (4) there is formed a lug (5) used as an iris hook.

4. A hand tip according to one of the preceding Claims, characterised in that the projecting end of the inner tube (1) is rectilinear.

5. A hand tip according to one of Claims 1 to 3, characterised in that the projecting end of the inner tube is formed like an elbow.

6. A unit consisting of two or more hand tips according to the precharacterising clause of Claim 1 for performing a lenticular cataract operation, with the aspirating aperture at the front end of the inner tube having a variable size, characterised in that the hand tip having the smallest aspirating aperture (3) comprises a polished surface (8) on the side opposite said aspirating aperture.

## Revendications

1. Embout manuel à double canule pour éliminer, par aspiration, des débris du cristallin lors de l'exécution d'opérations de la cataracte lenticulaire, la double canule se composant de deux petits tubes coaxiaux parmi les-quels le petit tube interne présente un bout fermé et un orifice d'aspiration ménagé latéralement, et l'extrémité antérieure du petit tube externe est reliée au petit tube interne à distance du bout, et comporte deux orifices de rinçage ménagés latéralement, caractérisé par le fait qu'une surface polie (8) est prévue du côté opposé à l'orifice d'aspiration (3).

2. Embout manuel selon la revendication 1, caractéri - sé par le fait que la surface polie (8) est formée par une poudre de diamant agglomérée par frittage.

3. Embout manuel selon la revendication 1 ou 2, caractérisé par le fait qu'une butée (5), servant d'ardillon agissant sur l'iris, est façonnée du côté du petit tube externe (2) qui est opposé aux deux orifices de rinçage (4).

4. Embout manuel selon l'une des revendications précédentes, caractérisé par le fait que l'extrémité saillante du petit tube interne (1 ) est de réalisation rectiligne.

5. Embout manuel selon l'une des revendications 1 à 3, caractérisé par le fait que l'extrémité saillante du petit tube interne (1) est de réalisation cintrée.

6. Ensemble unitaire comprenant deux ou plus de deux embouts manuels selon le préambule de la revendication 1, pour l'exécution d'une opération de la cataracte lenticulaire, l'orifice d'aspiration ménagé à l'extrémité antérieure des petits tubes internes présentant une taille différente, caractérisé par le fait que l'embout manuel muni de l'orifice d'aspiration (3) le plus petit comporte une surface polie (8) du côté opposé audit orifice d'aspiration.

# FIG. 1

# FIG. 2